(19) Europäisches Patentamt
European Patent Office
Office européen des brevets

(11) Veröffentlichungsnummer: **0 253 293**
**B1**

(12) # EUROPÄISCHE PATENTSCHRIFT

(45) Veröffentlichungstag der Patentschrift: **28.02.90**

(51) Int. Cl.[5]: **C 07 C 237/36**, C 07 C 279/14, C 07 C 231/12, A 61 K 31/16

(21) Anmeldenummer: **87109865.3**

(22) Anmeldetag: **08.07.87**

(54) **Neue Guanidiniumasparaginate.**

(30) Priorität: **11.07.86 CH 2795/86**

(43) Veröffentlichungstag der Anmeldung: **20.01.88 Patentblatt 88/03**

(45) Bekanntmachung des Hinweises auf die Patenterteilung: **28.02.90 Patentblatt 90/09**

(84) Bennante Vertragsstaaten: **AT BE CH DE ES FR GB GR IT LI LU NL SE**

(56) Entgegenhaltungen: **DE-A-2 819 898**

(73) Patentinhaber: **CIBA-GEIGY AG**
**Klybeckstrasse 141**
**CH-4002 Basel (CH)**

(72) Erfinder: **Baschang, Gerhard, Dr.**
**Bückenweg 7**
**CH-4126 Bettingen (CH)**
Erfinder: **Sallmann, Alfred, Dr.**
**Joachimsackerstrasse 12**
**CH-4103 Bottmingen (CH)**

(74) Vertreter: **Zumstein, Fritz, Dr.**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 253 293 B1

LIBERGRAF, STOCKHOLM 1990

**Beschreibung**

Die Erfindung betrifft neue Guanidiniumasparaginate, insbesondere das L-Argininsalz der N-(D)-{[2-(2,6-Dichlorphenylamino)phenyl]acetyl}asparaginsäure, dargestellt durch die Formel

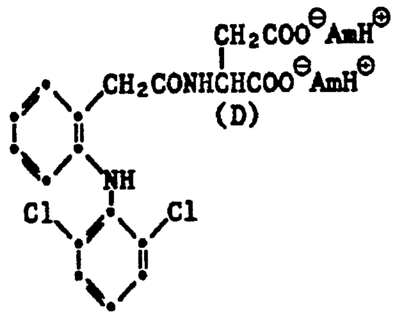

(I),

worin $AmH^{\oplus}$ das von L-Arginin Am der Formel

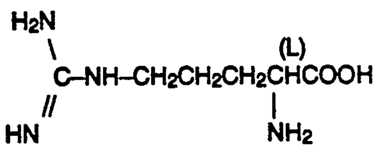

(II)

abgeleitete Kation darstellt, und seine Hydrate, Verfahren zur Herstellung der erfindungsgemässen Verbindungen, diese enthaltende pharmazeutische Präparate und ihre Verwendung.

Im Stand der Technik ist bekannt, dass die der Verbindung der Formel I zugrundeliegende D-{[2-(2,6-Dichlorphenylamino)phenyl]acetyl}asparaginsäure der Formel

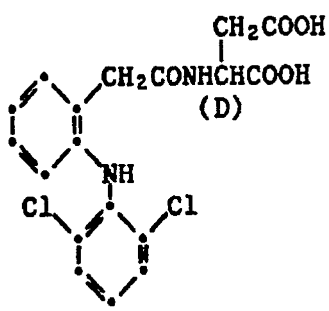

(III)

und ihre pharmazeutisch verwendbaren Salze, d.h. ihre Alkalimetall-, Erdalkalimetall- und Ammoniumsalze sowie Salze mit organischen Basen bei verhältnismässig guter gastrointestinaler Verträglichkeit entzündungshemmende und analgetische Eigenschaften aufweisen. Im Stand der Technik beschrieben ist jedoch lediglich die D–N–{[2-(2,6-Dichlorphenylamino)phenyl]acetyl}-asparaginsäure und deren Mononatriumsalz-Monohydrat.

Die Säure der Formel III und ihr Mononatriumsalz-Monohydrat haben jedoch empfindliche Nachteile bezüglich ihrer Verwendung als Arzneimittelwirkstoff. So ist die freie Säure in Wasser praktisch unlöslich und deshalb nicht für alle, insbesondere nicht für flüssige, galenische Formulierungen geeignet. Sie besitzt zudem einen bitteren Geschmack. Das Salz ist ebenfalls sehr wenig wasserlöslich. Seine wässrige Lösung hat zudem einen physiologisch ungünstigen pH-Wert und einen äusserst unangenehmen bitteren Geschmack, Eigenschaften durch die die Verwendbarkeit auch des Salzes als Arzneimittelwirkstoff stark eingeschränkt wird. Die schlechte Wasserlöslichkeit und der physiologisch ungünstige pH-Wert der wässrigen Lösung verunmöglicht die Herstellung flüssiger Applikationsformen, wie oralen flüssigen Darreichungsformen, z. B. von Sirup- oder Tropfenformulierungen, und parenteralen flüssigen Applikationsformen, z. B. von Injektions- und Infusionslösungen. Der physiologisch ungünstige pH-Wert führt zudem zu Verträglichkeitsproblemen bei der Applikation über Suppositorien, während der nicht zu überdeckende bittere Geschmack den Spielraum des Galenikers bei der Formulierung oraler Darreichungsformen stark einengt und praktisch auf Schutzschichtformen, wie Kapseln, Lacktabletten und dergleichen reduziert mit deren als nachteilig bekannten verzögerten Wirkungseintritt.

Der Erfindung liegt die überraschende Feststellung zugrunde, dass das Salz der Formel I hervorragend wasserlöslich ist, und dass seine wässrigen Lösungen einen praktisch neutralen pH-Wert und einen angenehm

süssen Geschmack besitzen. Auf Grund dieser überraschenden Eigenschaften können die noch bestehenden Schwierigkeiten bezüglich der Applikation dieser Verbindungsgruppe ausgeschaltet werden.

So beträgt die Löslichkeit des N-D-{[2-(2,6-Dichlorphenylamino)phenyl]acetyl}asparaginatsäure-L-Argininsalztrihydrates bei 20°C etwa 25 Gew.-% und der pH-Wert einer 5,0 Gew.-%-igen wässrigen Lösung 6,90, wohingegen das genannte Mononatriumsalz-monohydrat dieser Säure nur zu 0,5 Gew.-% wasserlöslich ist und der pH einer solchen Lösung den physiologisch ungünstigen Wert von 4,62 aufweist. Beide Eigenschaften, die stark verbesserte Wasserlöslichkeit und der praktisch neutrale pH-Wert wässriger Lösungen, des erfindungsgemäss bereitgestellten L-Argininsalzes sind im Hinblick auf flüssige Applikationsformen, wie die genannten von grosser Bedeutung. Der angenehme Geschmack ist äusserst vorteilhaft für sämtliche oralen Darreichungsformen.

Das L-Argininsalz der Formel I weist wertvolle pharmakologische Eigenschaften auf, die denen des bekannten Salzes praktisch entsprechen.

Insbesondere zeigt es eine potente entzündungshemmende Wirkung in chronischen und subchronischen Entzündungsmodellen, wie der experimentellen Adjuvans-Arthritis der Ratte. In diesem Modell beträgt die $ED_{40}$ für das D-N-{[2-(2,6-Dichlorphenylamino)phenyl]-acetyl}asparaginsäure-di-L-Argininsalztrihydrat 0,5 mg/kg p.o. Die Verbindung zeichnet sich zudem durch eine ausserordentlich gute gastrointestinale Verträglichkeit aus. So wurde im akuten Rattenulcus-Modell bis zur höchsten geprüften Dosis von 160 mg/kg p.o. keine Ulcerogenität nachgewiesen. Auch im 10-Tage-Ulcustest wurde bei täglicher Gabe von 160 mg/kg p.o. keine Mortalität festgestellt. Der gastrointestinale Blutverlust war bei dieser Dosierung gegenüber Kontrolltieren nur geringfügig erhöht.

Diese Ergebnisse zeigen, dass das neue Salz eine überraschend hohe therapeutische Breite besitzt, die es positiv gegenüber klassischen Entzündungshemmern abhebt.

Die Erfindung betrifft ebenso ein auf an sich bekannten Methoden beruhendes Verfahren zur Herstellung der Guanidiniumasparaginate der Formel I, dadurch gekennzeichnet, dass man L-Arginin, d.h. die Verbindung der Formel

```
H2N
   \                 (L)
    C-NH-CH2CH2CH2CHCOOH                (II)
   //                |
HN                  NH2
```

mit N-(D)-{[2-(2,6-Dichlorphenylamino)phenyl]acetyl}asparaginsäure der Formel

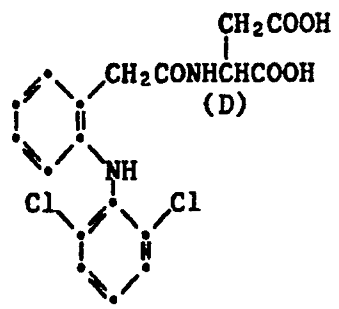

(III)

umsetzt.

Die Umsetzung erfolgt in üblicher Weise, beispielsweise in wässriger oder wasserhaltiger Lösung, d.h. in einem Gemisch von Wasser und einem mit Wasser mischbaren Lösungsmittel, wie einem Niederalkanol oder Diniederalkylketon, z. B. Methanol, Äthanol oder Aceton. Bei Durchführung der Umsetzung in wässriger Lösung wird das Reaktionsprodukt vorteilhaft durch Lyophilisierung (eine Form der Gefriertrocknung) isoliert.

Die Erfindung betrifft ebenfalls die Verwendung der salzartigen Verbindung der Formel I als pharmakologisches, in erster Linie entzündungshemmendes Mittel zur Behandlung von chronischen und subchronischen entzündlichen Erkrankungen, wie Entzündungen traumatischer und/oder degenerativer Äthiologie, z. B. von Arthritis bzw. Polyarthritis. Dabei kann man sie, vorzugsweise in Form von pharmazeutischen Präparaten, in einem Verfahren zur prophylaktischen und/oder therapeutischen Behandlung des tierischen und menschlichen Körpers, insbesondere zur Behandlung von chronischen und subchronischen Entzündungszuständen, wie arthritischer Krankheitsbilder, verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen z. B. für Warmblüter mit einem Körpergewicht von etwa 70 kg, je nach Art der Erkrankung, individuellem Zustand und Alter, vorzugsweise zwischen etwa 100 und 500 mg, insbesondere zwischen etwa 200 mg und etwa 500 mg, und speziell etwa bei 250 mg bis etwa 400 mg.

Die Erfindung betrifft im weiteren pharmazeutische Präparate, die die Verbindung der Formel I als Wirkstoff enthalten, sowie Verfahren zu deren Herstellung.

Bei den erfindungsgemässen pharmazeutischen Präparaten handelt es sich um solche zur parenteralen oder enteralen, wie peroralen oder rektalen, aber auch sublingualen, Verabreichung an Warmblüter. Diese enthalten vorzugsweise von etwa 20 mg bis etwa 200 mg, insbesondere von etwa 50 mg bis etwa 150 mg einer Verbindung der Formel I zusammen mit pharmazeutisch verwendbaren Trägerstoffen.

Für parenterale Applikation eignen sich auf Grund der ausgezeichneten Wasserlöslichkeit der salzartigen Verbindung der Formel I insbesondere wässrige Lösungen, die beispielsweise in Dosiseinheitsform, wie in Ampullen oder Vials, abgefüllt werden. Wässrige Lösungen enthalten z. B. von etwa 1,0, vorzugsweise 2,0 % (g/100 ml) bis zur Sättigung, insbesondere bis etwa 20,0 vorzugsweise 10,0 % (g/100 ml) Wirkstoff.

Für orale Applikation sind beispielsweise feste Darreichungsformen, vorzugsweise solche in Dosiseinheitsform, wie Tabletten, ferner Lacktabletten oder Kapseln, z. B. Gelatinesteckkapseln oder geschlossene Gelatinekapseln, vor allem aber zu flüssigen oralen Darreichungsformen auflösbare Formulierungen, wie wasserlösliche Pulver bzw. Brausepulver, vorzugsweise in Einzeldosisbeutelchen (Sachets), Brausetabletten oder rekonstituierbare Trokkensirupe, bzw. flüssige orale Darreichungsformen, wie Tropfen, Mixturen, Säfte, Sirupe, Elixiere und dergleichen, und für rektale Applikation beispielsweise Suppositorien geeignet. Feste Darreichungsformen enthalten etwa zwischen 2 und 80 Gew.-%, Tabletten, Lacktabletten, z. B. zwischen etwa 10 und 60 Gew.-%, Kapseln z. B. zwischen etwa 20 und 80 Gew.-% und Suppositorien z. B. zwischen etwa 2 und 25 Gew.-% Wirkstoff. Flüssige orale Darreichungsformen enthalten z. B. von etwa 1,0, vorzugsweise 2,0 % (g/100 ml) bis zur Sättigung, insbesondere bis etwa 20,0 vorzugsweise 10,0 (g/100 ml) Wirkstoff.

Die erfindungsgemässen pharmazeutischen Präparate können auf an sich bekannte Weise hergestellt werden, indem man die Wirkstoffkomponente mit üblichen pharmazeutischen Hilfsstoffen vermischt.

Zur parenteralen Verabreichung durch Injektion bzw. Infusion eignen sich, wie erwähnt, in erster Linie isotonische wässrige Lösungen, die neben Zusätzen zur Regulierung des osmotischen Druckes, wie Natriumchlorid, stabilisierende Mittel, wie Schwermetall-Komplexbildner, Antioxidantien und/oder Pufferlösungen enthalten können.

Oral anwendbare feste Darreichungsformen, wie die genannten, können durch übliche Misch-, Granulier- oder Dragierverhalten hergestellt werden. So können die Wirkstoffe mit festen Trägerstoffen gemischt werden, eine entstehende Mischung kann granuliert werden, und die Mischung oder das Granulat kann, gewünschten- oder nötigenfalls nach Zugabe geeigneter Hilfsmittel, zu Tabletten oder Dragéekernen verarbeitet werden. Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, Bindemittel, wie Stärkekleister auf der Basis z. B. von Mais-, Weizen-, Reis- oder Kartoffelstärke, Gelatine, Methylcellulose, Hydroxypropylmethylcellulose, Hydroxypropylcellulose, und/oder Polyvinylpyrrolidon, und/oder, wenn erwünscht, Sprengmittel, wie die genannten Stärken, ferner Carboxymethylstärke, quervernetztes Polyvinylpyrrolidon, Agar, Alginsäure oder ein Salz davon, wie Natriumalginat, oder niedermolekulare Carboxymethylcellulose. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol.

Dragéekerne werden mit geeigenten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder wässrige Dispersionen von Äthylacrylat/Methylmethacrylat-Copolymeren verwendet. Zur Herstellung von Magensaftresistenten Überzügen verwendet man Lösungen von geeigneten Cellulosepräparaten, wie Acetylcellulosephthalat oder Hydroxypropylmethylcellulosephthalat, oder wässrige Dispersionen von z. B. Methacrylsäure/Methacrylat-Copolymeren. Den Tabletten oder Überzügen können Farbstoffe oder Pigmente, z. B. zur Identifizierung oder zur Kennzeichnung verschiedener Wirkstoffdosierungen beigefügt werden.

Steckkapseln aus Gelatine sowie weiche, geschlossene Kapseln aus Gelatine und einem Weichmacher, wie Glycerin oder Sorbit können den Wirkstoff als Pulver oder Granulat, z. B. im Gemisch mit Füllstoffen, wie Cellulose oder Lactose, Bindemitteln, wie Stärken, und/oder Schmiermitteln, wie Talk oder Magnesiumstearat, und erforderlichenfalls übliche Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Zu flüssigen oralen Darreichungsformen auflösbare Formulierungen, wie wasserlösliche Pulver bzw. Brausepulver, vorzugsweise in Einzeldosisbeutelchen (Sachets), Brausetabletten oder rekonstituierbare Trockensirupe, basieren auf der Reaktion einer pharmazeutisch verwendbaren organischen Säure mit einer Alkalimetall- oder Erdalkalimetallcarbonatverbindung, wobei Kohlendioxyd freigesetzt wird. Als pharmazeutisch verwendbare organische Säuren kommen beispielsweise Zitronensäure, Weinsäure, Fumarsäure, Maleinsäure, Ascorbinsäure und ähnliche gegebenenfalls hydroxylierte aliphatische Dicarbonsäuren, als Alkalimetall- bzw. Erdalkalicarbonatverbindung beispielsweise Natriumhydrogencarbonat, Natriumcarbonat, Calcium- oder Magnesiumcarbonat oder Calciumhydrogencarbonat in Betracht. Weitere galenische Bestandteile entsprechen im wesentlichen denen von Tabletten. Zusätzlich werden üblicherweise Farb- und/oder Aromastoffe sowie Konservierungsmittel zugeschlagen.

Flüssige orale Darreichungsformen, wie Tropfen, Mixturen und Säfte, sind ihrer physikalischen Erscheinungsform nach vorzugsweise Lösungen, können aber auch als rekonstitutierbare Festformen, wie Trockensirupe, vorliegen.

Lösungen sind Zubereitungen, die durch Auflösen des Wirkstoffes in Wasser oder einem geeigneten wasserhaltigen Lösungsmittelgemisch erhalten werden.

Sirupe sind dickflüssige wässrige oder wasserhaltige Lösungen, die Zucker oder Zuckeralkohole (zum Beispiel Sorbitol) in hoher Konzentration enthalten.

Elixiere sind wässrig-alkoholische gesüsste Lösungen. Ihr hoher Gehalt an Ethanol und anderen Lösungsmitteln, wie Glykolen, ermöglicht es, schwer wasserlösliche Wirkstoffe in Form klarer Lösungen zu verabreichen.

Bei Trockensirupen handelt es sich um Präparate, die als Pulver oder Granulat alle Bestandteile der fertigen Zubereitung enthalten, so dass vor Gebrauch nur noch die erforderliche Wassermenge zur Auflösung hinzuzufügen ist. Der rekonstituierte Sirup bleibt während der vom Hersteller angegebenen Verbrauchsfrist stabil.

Die genannten flüssigen Darreichungsformen können gesüsst, aromatisiert und/oder gefärbt sein.

Suppositorien bestehend aus einer Kombination des Wirkstoffs mit einer Suppositoriengrundmasse eignen sich, z. B. natürliche oder synthetische Glycerinester, Paraffinkohlenwasserstoff, Polyäthylenglykole oder höhere Alkanole oder Mischungen davon. Ferner können auch Gelatine-Rektalkapseln verwendet werden, die eine Kombination des Wirkstoffs mit einer Grundmasse enthalten; als Grundmasse kommen z. B. flüssige Triglyceride, Polyäthylenglykole oder Paraffinkohlenwasserstoffe zusammen mit Suspensionsstabilisatoren, wie Wachsen und anderen Quellmitteln in Frage.

Die nachstehenden Beispiele dienen lediglich zur Illustration der Erfindung und sollen diese in keiner Weise einschränken. Temperaturen sind in Celciusgraden angegeben.

**Beispiel 1**

Zu einer Lösung von 46,64 g L-Arginin in 300 ml Wasser werden bei Raumtemperatur unter Rühren 55,0 g D-N-{[2-(2,6-Dichlorphenylamino)phenyl]acetyl}-asparaginsäure zugesetzt. Man rührt 30 Minuten nach, friert die klare Lösung im Aceton/Trockeneis-Bad ein und lyophilisiert anschliessend. Der Rückstand, das D-{[2-(2,6-Dichlorphenylamino)phenyl]acetyl}asparaginsäure-L-Argininsalz-trihydrat ist ein weisses, amorphes Pulver vom Smp. 155 - 165° (Zers.); Löslichkeit: 25 Gew.-% in Wasser von 20°C.

**Beispiel 2**

Eine Injektionslösung in Dosiseinheitsform, enthaltend 150 mg N-(D)-{[2-(2,6-Dichlorphenylamino)phenyl]acetyl}asparaginsäure-L-Argininsalz-trihydrat als Wirkstoff, kann z. B. folgendermassen hergestellt werden:

**Zusammensetzung** (für 1000 Dosiseinheiten)

| | |
|---|---|
| Wirkstoff | 150,00 g |
| Natriumchlorid | 22,50 g |
| Wasser | ad 2500,00 ml |

Der Wirkstoff wird in 1250 ml Wasser eingetragen und unter lebhaftem Rühren gelöst. Man filtriert durch ein Mikrofilter, verdünnt unter Rühren mit einer Lösung von 22,5 g Natriumchlorid in 1000,0 ml Wasser, füllt Wasser auf 2500 ml auf und verschliesst unter sterilen Bedingungen je 2,50 ml der Lösung in Glasampullen.

**Beispiel 3**

Tabletten, enthaltend 380 mg N-(D)-{[2-(2,6-Dichlorphenylamino)phenyl]acetyl}asparaginsäure-L-Argininsalz-trihydrat als Wirkstoff, können wie folgt hergestellt werden

**Zusammensetzung** (für 1000 Tabletten)

| | |
|---|---|
| Wirkstoff | 380,00 g |
| Lactose | 25,00 g |
| Maisstärke | 5,00 g |
| Talk | 5,50 g |
| Calciumstearat | 1,50 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 400 mg) verpresst. Gewicht der Lacktablette: ca. 415 mg.

**Beispiel 4**

Tabletten, enthaltend 50 mg N-(D)-{[2-(2,6-Dichlorphenylamino)phenyl]acetyl}asparaginsäure-L-Argininsalz-trihydrat als Wirkstoff, können wie folgt hergestellt werden:

**Zusammensetzung** (für 1000 Tabletten)

| | |
|---|---|
| Wirkstoff | 50,0 g |
| Lactose | 155,0 g |
| Kartoffelstärke | 150,0 g |
| Gelatine | 25,0 g |
| Talk | 50,0 g |
| Magnesiumstearat | 15,0 g |
| Siliciumdioxid (hochdispers) | 10,0 g |
| Äthanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 600 mg Gewicht uhd dem oben angegebenen Wirkstoffgehalt, die gewünschtenfalls mit Teilkerben zur feineren Anpassung der Dosierung versehen sein können.

**Beispiel 5**

Lacktabletten, enthaltend 100 mg N-(D)-{[2-(2,6-Dichlorphenylamino)phenyl]acetyl}asparaginsäure-L-Argininsalz-trihydrat als Wirkstoff, können wie folgt hergestellt werden:

**Zusammensetzung** (für 1000 Tabletten)

| | |
|---|---|
| Wirkstoff | 100,00 g |
| Lactose | 90,00 g |
| Maisstärke | 50,00 g |
| Talk | 7,50 g |
| Calciumstearat | 3,50 g |
| Hydroxypropyl-methylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 300 mg.

**Beispiel 6**

Gelatinesteckkapseln, enthaltend 350 mg N-(D)-{[2-(2,6-Dichlorphenylamino)phenyl]acetyl}asparaginsäure-L-Argininsalz-trihydrat als Wirkstoff, können wie folgt hergestellt werden:

**Zusammensetzung** (für 1000 Kapseln)

| | |
|---|---|
| Wirkstoff | 350,00 g |
| mikrokristalline Cellulose | 30,00 g |
| Natriumlaurylsulfat | 2,00 g |
| Magnesiumstearat | 8,00 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse 0 (elongated) abgefüllt.

**Beispiel 7**

Brausetabletten, enthaltend 100 mg N-(D)-{[2-(2,6-Dichlorphenylamino)phenyl]acetyl}asparaginsäure-L-Argininsalztrihydrat als Wirkstoff, können wie folgt hergestellt werden:

**Zusammensetzung** (für 1000 Einheiten)

| | |
|---|---|
| Wirkstoff | 100,00 g |
| Zitronensäure, wasserfrei | 500,00 g |
| Natriumhydrogencarbonat, granulös | 525,00 g |
| Natriumcarbonat, wasserfrei | 375,00 g |
| Orangenaroma, natürlich | 5,00 g |
| Natriumbenzoat | 95,00 g |

Die Komponenten werden innig vermahlen und in einer Hochdruck-Tablettenpresse zu Tabletten von je 1,6 g Gewicht verpresst.

**Beispiel 8**

Einzeldosisbeutelchen (Sachets), enthaltend 100 mg N-(D)-{[2-(2,6-Dichlorphenylamino)phenyl]acetyl}asparaginsäure-L-Argininsalz-trihydrat als Wirkstoff, können wie folgt hergestellt werden:

**Zusammensetzung** (für 1000 Einheiten)

| | |
|---|---|
| Wirkstoff | 100,00 g |
| Zitronensäure, wasserfrei | 140,00 g |
| Zitronensaft, gefriergetrocknet | 90,00 g |
| Zitronenaromen, natürlich | 1,00 g |
| Natriumcarbonat | 30,00 g |
| Natriumhydrogencarbonat | 55,00 g |
| Natriumsaccharin | 5,00 g |

Die Komponenten werden, gegebenenfalls nach Granulierung mit Äthanol, in Polyäthylenbeutelchen, enthaltend je 0,65 g Formulat abgefüllt.

**Patentansprüche** für die Vertragsstaaten: BE, CH, DE, FR, GB, IT, LU, NL, SE

1. Das L-Argininsalz der N-(D)-{[2-(2,6-Dichlorphenylamino)phenyl]acetyl}asparaginsäure, dargestellt durch die Formel

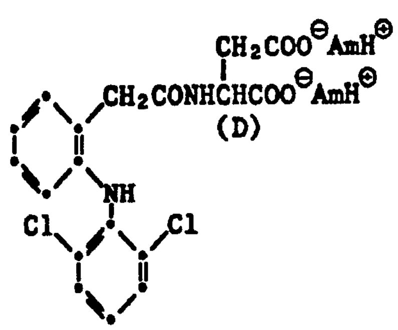

(I),

worin $AmH^{\oplus}$ das von L-Arginin Am der Formel

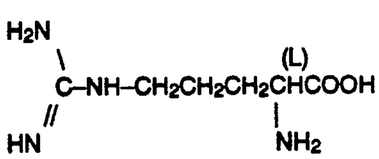

(II)

abgeleitete Kation darstellt, und ihre Hydrate.

2. N-(D)-{[2-(2,6-dichlorphenylamino)phenyl]acetyl}asparginsäure-L-Argininsalz gemäß Anspruch 1 in Form des Trihydrates.

3. Verfahren zur Herstellung des L-Argininsalzes der N-(D)-{[2-(2,6-Dichlorphenylamino)phenyl]acetyl}asparaginsäure, dargestellt durch die Formel

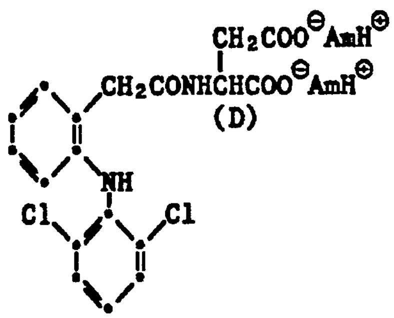

(I),

worin $AmH^{\oplus}$ das von L-Arginin Am der Formel

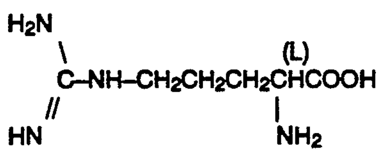

(II)

abgeleitete Kation darstellt, und ihrer Hydrate, dadurch gekennzeichnet, dass man L-Arginin mit N-(D)-{[2-(2,6-Dichlorphenylamino)phenyl]acetyl}-asparaginsäure umsetzt.

4. Eine Verbindung gemäss einem der Ansprüche 1 und 2 zur Anwendung in einem Verfahren zur therapeutischen Behandlung des menschlichen oder tierischen Körpers.

5. Pharmazeutische Präparate, enthaltend eine Verbindung gemäss einem der Ansprüche 1, 2 und 4 neben üblichen pharmazeutischen Hilfsstoffen.

6. Pharmazeutische Präparate gemäss einem Anspruch 5 in flüssiger Form zur parenteralen Applikation, wie Injektions- oder Infusionslösungen.

7. Pharmazeutische Präparate gemäss Anspruch 5 zur enteralen Applikation, wie feste oder flüssige bzw. auflösbare orale oder feste rektale Darreichungsformen.

8. Pharmazeutische Präparate gemäss Anspruch 7 in flüssiger Darreichungsform zur oralen Applikation, wie Tropfen, Mixturen, Säfte, Elixiere und Sirupe bzw. in Form von wasserlöslichen Pulvern, Brausetabletten oder rekonstituierbaren Trockensirupen.

9. Pharmazeutische Präparate gemäss Anspruch 8 in zu flüssigen oralen Darreichungsformen auflösbarer Formulierung, wie wasserlösliche Pulver bzw. Brausepulver, vorzugsweise in Einzeldosisbeutelchen, Brausetabletten oder rekonstituierbare Trockensirupe.

10. Wässrige bzw. wasserhaltige Lösungen gemäss einem der Ansprüche 5 - 8.

11. Pharmazeutische Präparate gemäss Anspruch 7 in fester Darreichungsform zur oralen oder rektalen Applikation, wie Tabletten oder Kapseln bzw. Suppositorien.

12. Verwendung einer Verbindung gemäss einem der Ansprüche 1, 2 und 4 zur Herstellung entzündungshemmender Arzneimittel.

**Patentansprüche** für die Vertragsstaaten: AT, ES, GR

1. Verfahren zur Herstellung des L-Argininsalzes der N-(D)-{[2-(2,6-Dichlorphenylamino)phenyl]acetyl}asparaginsäure, dargestellt durch die Formel

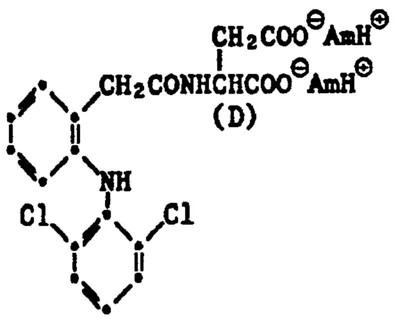

(I),

worin AmH$^{\oplus}$ das von L-Arginin Am der Formel

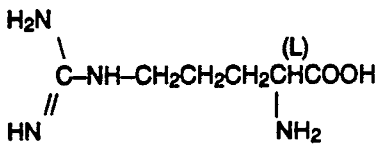

(II)

abgeleitete Kation darstellt, und ihrer Hydrate, dadurch gekennzeichnet, dass man L-Arginin mit N-(D)-{[2-(2,6-Dichlorphenylamino)phenyl]acetyl}asparaginsäure umsetzt.

2. Verfahren gemäss Anspruch 1, dadurch gekennzeichnet, dass man N-(D)-{[2-(2,6-dichlorphenylamino)phenyl]acetyl}asparginsäure-L-Argininsalz in Form des Trihydrates herstellt.

3. Verfahren zur Herstellung pharmazeutischer Präparate, dadurch gekennzeichnet, dass man eine Verbindung, erhältlich gemäss einem der Ansprüche 1 und 2 mit üblichen pharmazeutischen Hilfsstoffen herstellt.

4. Verfahren gemäss Anspruch 3 zur Herstellung pharmazeutischer Präparate in flüssiger Form zur parenteralen Applikation, wie Injektions- oder Infusionslösungen.

5. Verfahren gemäss Anspruch 3 zur Herstellung pharmazeutischer Präparate zur enteralen Applikation, wie feste oder flüssige bzw. auflösbare orale oder feste rektale Darreichungsformen.

6. Verfahren gemäss Anspruch 3 zur Herstellung pharmazeutischer Präparate in flüssiger Darreichungsform zur oralen Applikation, wie Tropfen, Mixturen, Säfte, Elixiere und Sirupe bzw. in zu flüssigen oralen Darreichungsformen auflösbarer Formulierung, wie wasserlösliche Pulver bzw. Brausepulver, vorzugsweise in Einzeldosisbeutelchen, Brausetabletten oder rekonstituierbare Trockensirupe.

7. Verfahren gemäss Anspruch 3 zur Herstellung pharmazeutischer Präparate in fester Darreichungsform zur oralen oder rektalen Applikation, wie Tabletten oder Kapseln bzw. Suppositorien.

8. Verwendung einer Verbindung gemäß Anspruch 1 oder 2 zur Herstellung entzündungshemmender Arzneimittel.

**Claims** for the Contracting States: BE, CH, DE, FR, GB, IT, LI, LU, NL, SE

1. The L-arginine salt of N-(D)-([2-(2, 6-dichlorophenylamino)phenyl]acetyl}aspartic acid, represented by the formula

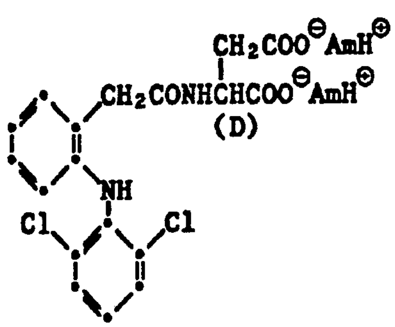

(I)

in which AmH$^{\oplus}$ represents the cation derived from L-arginine Am of the formula

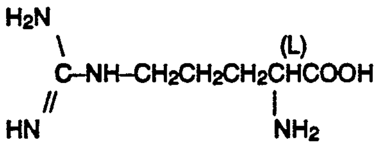

(II),

and its hydrates.

2. N-(D)-{[2-(2,6-dichlorophenylamino)phenyl]acetyl}aspartic acid L-arginine salt according to claim 1 in the form of the trihydrate.

3. A process for the manufacture of the L-arginine salt of N-(D)-{[2-(2,6-dichlorophenylamino)phenyl]acetyl}aspartic acid, represented by the formula

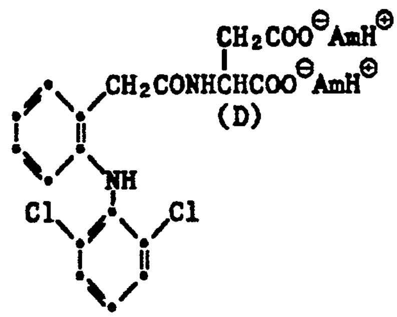

(I)

in which $AmH^{\oplus}$ represents the cation derived from L-arginine Am of the formula

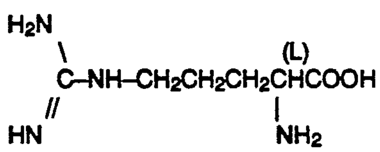

(II),

and its hydrates, characterised in that L-arginine is reacted with N-(D)-{[2-(2,6-dichlorophenylamino)phenyl]acetyl}aspartic acid.

4. A compound according to either of claims 1 and 2 for use in a method for the therapeutic treatment of the human or animal body.

5. Pharmaceutical preparations containing a compound according to any one of claims 1, 2 and 4 together with customary pharmaceutical adjuncts.

6. Pharmaceutical preparations according to claim 5 in liquid form for parenteral administration, such as injection solutions or infusion solutions.

7. Pharmaceutical preparations according to claim 5 for enteral administration, such as solid or liquid/dissoluble oral, or solid rectal forms of administration.

8. Pharmaceutical preparations according to claim 7 in liquid administration form for oral administration, such as drops, mixtures, juices, elixirs and syrups, or in the form of water-soluble powders, effervescent tablets or reconstitutable dry syrups.

9. Pharmaceutical preparations according to claim 8 formulated so that they can be dissolved to form liquid oral forms of administration, such as water-soluble powders and effervescent powders, preferably in single dose sachets, effervescent tablets or reconstitutable dry syrups.

10. Aqueous or water-containing solutions according to any one of claims 5 to 8.

11. Pharmaceutical preparations according to claim 7 in solid administration form for oral or rectal administration, such as tablets or capsules and suppositories.

12. The use of a compound according to any one of claims 1, 2 and 4 for the manufacture of inflammation-inhibiting medicaments.

**Claims** for the Contracting States: AT, ES, GR

1. A process for the manufacture of the L-arginine salt of N-(D)-{[2-(2,6-dichlorophenylamino)phenyl]acetyl}aspartic acid, represented by the formula

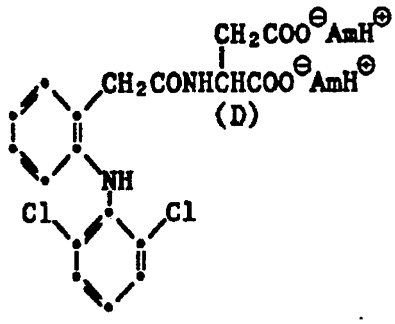

(I)

in which $AmH^{\oplus}$ represents the cation derived from L-arginine Am of the formula

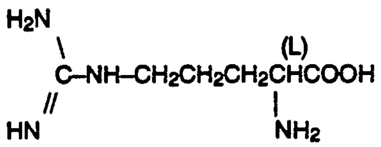

(II),

and its hydrates, characterised in that L-arginine is reacted with N-(D)-{[2-(2,6-dichlorophenylamino)phenyl]acetyl}aspartic acid.

2. A process according to claim 1, characterised in that N-(D)-{[2-(2,6-dichlorophenylamino)phenyl]acetyl}aspartic acid L-arginine salt in the form of the trihydrate is manufactured.

3. A process for the manufacture of pharmaceutical preparations, characterised in that a compound obtainable according to either of claims 1 and 2 is manufactured together with customary pharmaceutical adjuncts.

4. A process according to claim 3 for the manufacture of pharmaceutical preparations in liquid form for parenteral administration, such as injection solutions or infusion solutions.

5. A process according to claim 3 for the manufacture of pharmaceutical preparations for enteral administration, such as solid or liquid/dissoluble oral, or solid rectal forms of administration.

6. A process according to claim 3 for the manufacture of pharmaceutical preparations in liquid administration form for oral administration, such as drops, mixtures, juices, elixirs and syrups, or formulated so that they can be dissolved to form liquid oral forms of administration, such as water-soluble powders and effervescent powders, preferably in single dose sachets, effervescent tablets or reconstitutable dry syrups.

7. A process according to claim 3 for the manufacture of pharmaceutical preparations in solid administration form for oral or rectal administration, such as tablets or capsules and suppositories.

8. The use of a compound according to claim 1 or 2 for the manufacture of inflammation-inhibiting medicaments.

**Revendications** pour les Etats Contractants: BE, CH, DE, FR, GB, IT, LI, LU, NL, SE

1. Le sel de L-arginine de l'acide N-(D)-{[2-(2,6-dichlorophénylamino)-phényl]-acétyl}-aspartique répondant à la formule

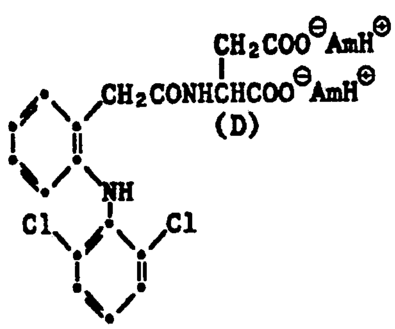

(I),

dans laquelle AmH$^{\oplus}$ représente le cation dérivé de la L-arginine Am de formule

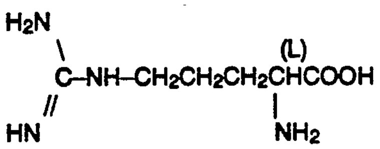

(II)

et ses hydrates.

2. Le sel de L-arginine de l'acide N-(D)-{[2-(2,6-dichlorophénylamino)-phényl]-acétyl}-aspartique selon la revendication 1 à l'état de trihydrate.

3. Procédé de préparation du sel de L-arginine de l'acide N-(D)-{[2-(2,6-dichlorophénylamino)-phényl]-acétyl}-aspartique répondant à la formule

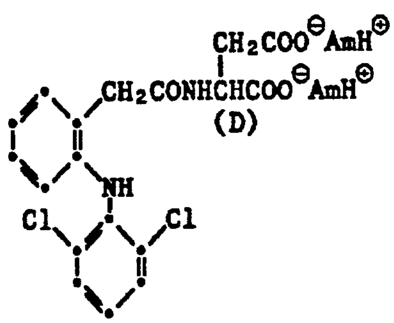

(I),

dans laquelle AmH$^{\oplus}$ représente le cation dérivé de la L-arginine Am de formule

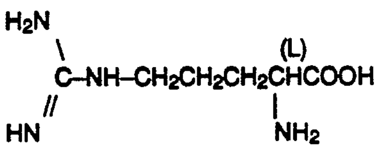

(II)

et de ses hydrates, caractérisé en ce que l'on fait réagir la L-arginine avec l'acide N-(D)-{[2-(2,6-dichlorophénylamino)-phényl]-acétyl}-aspartique.

4. Un composé selon l'une des revendications 1 et 2 pour l'utilisation dans un procédé pour le traitement thérapeutique de l'organisme humain ou animal.

5. Compositions pharmaceutiques contenant un composé selon l'une des revendications 1, 2 et 4 avec des produits auxiliaires pharmaceutiques usuels.

6. Compositions pharmaceutiques selon la revendication 5, à l'état liquide pour l'administration parentérale, par exemple solutions pour injections ou pour perfusions.

7. Compositions pharmaceutiques selon la revendication 5 pour l'administration entérale, par exemple formes d'administration orales solides ou liquides ou solubles ou formes d'administration rectales solides.

8. Compositions pharmaceutiques selon la revendication 7, à l'état de formes d'administrations liquides pour l'administration orale telles que gouttes, mixtures, breuvages, élixirs et sirops ou à l'état de poudres solubles dans l'eau, comprimés effervescents ou sirops secs reconstituables.

9. Compositions pharmaceutiques selon la revendication 8, donnant par dissolution des formes d'administration orales liquides, par exemple poudres ou poudres effervescentes solubles dans l'eau, de préférence dans des sachets-doses individuels, comprimés effervescents ou sirops secs reconstituables.

10. Solutions aqueuses ou contenant de l'eau selon l'une des revendications 5 à 8.

11. Compositions pharmaceutiques selon la revendication 7, à l'état de formes d'administration solides pour l'administration orale ou rectale, par exemple de comprimés ou capsules ou de suppositoires.

12. Utilisation d'un composé selon l'une des revendications 1, 2 et 4 pour la préparation de médicaments anti-inflammatoires.

**Revendications** pour les Etats Contractants: AT, ES, GR

1. Procédé de préparation du sel de L-arginine de l'acide N-(D)-{[2-(2,6-dichlorophénylamino)-phényl]-acétyl}-aspartique répondant à la formule

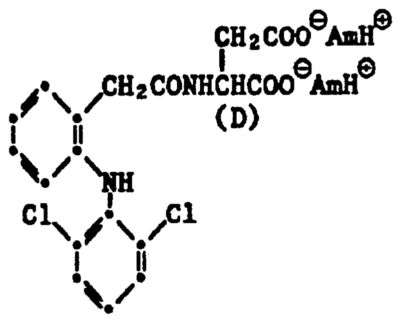

(I),

dans laquelle $AmH^{\oplus}$ représente le cation dérivé de la L-arginine Am de formule

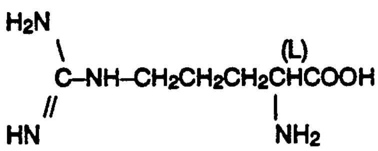

(II)

et de ses hydrates, caractérisé en ce que l'on fait réagir la L-arginine avec l'acide N-(D)-{[2-(2,6-dichlorophénylamino)-phényl]-acétyl}-aspartique.

2. Procédé selon la revendication 1, caractérisé en ce que l'on prépare le sel de L-arginine de l'acide N-(D)-{[2-(2,6-dichlorophénylamino)-phényl]-acétyl}-aspartique à l'état de trihydrate.

3. Procédé de préparation de compositions pharmaceutiques, caractérisé en ce que l'on combine un composé obtenu selon l'une des revendications 1 et 2 avec des produits auxiliaires pharmaceutiques usuels.

4. Procédé selon la revendication 3, pour la préparation de compositions pharmaceutiques à l'état liquide pour l'administration parentérale, par exemple des solutions pour injections ou pour perfusions.

5. Procédé selon la revendication 3, pour la préparation de compositions pharmaceutiques pour l'administration entérale, par exemple des formes d'administration orales solides ou liquides ou solubles ou des formes d'administration rectales solides.

6. Procédé selon la revendication 3, pour la préparation de compositions pharmaceutiques à l'état de formes d'administration liquides pour l'administration orale, par exemple des gouttes, des mixtures, des breuvages, des élixirs et des sirops, ou sous forme de compositions pouvant donner par dissolution des formes d'administration orales liquides, par exemple des poudres ou poudres effervescentes solubles dans l'eau, de préférence dans des sachets-doses individuels, des comprimés effervescents ou des sirops secs reconstituables.

7. Procédé selon la revendication 3, pour la préparation de compositions pharmaceutiques à l'état de formes d'administration solides pour l'administration orale ou rectale, par exemple des comprimés ou capsules ou des suppositoires.

8. Utilisation d'un composé selon la revendication 1 ou 2 pour la préparation de médicaments antiinflammatoires.